# EUROPEAN PATENT SPECIFICATION

(11) **EP 3 309 263 B1**
(45) Date of publication and mention of the grant of the patent: **25.12.2019**
(21) Application number: 17198700.1
(22) Date of filing: 26.06.2013
(51) Int. Cl.: C12Q 1/6886, A61K 31/454, A61K 31/506, A61K 31/519, G01N 33/50, G01N 33/574

(54) **BIOMARKERS ASSOCIATED WITH CDK INHIBITORS**
MIT CDK-INHIBITOREN ASSOZIIERTE BIOMARKER
BIOMARQUEURS ASSOCIÉS À DES INHIBITEURS DE CDK

(30) Priority: 09.07.2012 US 201261669534 P
(43) Date of publication of application: 18.04.2018
(62) Divisional of application: 13735529.3
(73) Proprietor: Novartis AG, 4056 Basel (CH); Dana-Farber Cancer Institute, Inc., Boston, MA 02215-5450 (US); Broad Institute, Boston, Massachusetts 02115 (US)
(72) Inventor: CAPONIGRO, Giordano, Cambridge, MA 02139 (US); DELACH, Scott, Cambridge, MA 02139 (US); GARRAWAY, Levi, Boston, MA 02215-5450 (US); JAGANI, Zainab, Cambridge, MA 02139 (US); KIM, Sunkyu, Cambridge, MA 02139 (US); KRYUKOV, Gregory, Cambridge, MA 02143 (US)
(74) Representative: Bucher, Tamaris Clare

(56) References cited:
- WO-A2-2009/150491
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]
- K. E. BERQUAM-VRIEZE ET AL: "Cell of origin strongly influences genetic selection in a mouse model of T-ALL", BLOOD, vol. 118, no. 17, 9 August 2011 (2011-08-09), pages 4646-4656, XP055271697, US ISSN: 0006-4971, DOI: 10.1182/blood-2011-03-343947

## Description

### FIELD OF THE INVENTION

The present invention relates to the field of pharmacogenomics, and the use of biomarkers useful in determining cell sensitivity to a cyclin dependant kinase inhibitor, methods of treatment and screening of compounds.

### BACKGROUND

Human cyclin D3 (CCND3) was first cloned in 1992, by screening for genes that would regulate the cell cycle, specifically in G1-S phase progression (Xiong et al., Genomics 1992, 13:575-584, Motokura et al., J. Biol. Chem. 1992, 267:20412-20415). It is one of three D-type cyclins (Cyclins D1, D2 and D3). It was discovered that CCND3 as a D-type cyclin would assemble with cyclin-dependent kinases CDK4 and CDK6 to form holoenzymes that facilitate progression through G1 of the cell cycle (Bates et al.,Oncogene 1994, 9:71-79) . Once late G1 is passed, CCND3 is no longer necessary for cell cycle progression (Sherr, Trends in Bio.Sci 1995, 20(5)187-190).

Structurally, CCND3 contains a cyclin domain and two PEST domains. PEST domains are used for ubiquitin mediated degradation and are found in proteins that the cell needs to regulate or turnover quickly. PEST domains are enriched in Proline (P), Glutamic Acid (E), Serine (S) and Threonine (T) residues and range from 12 to 60 amino acids in length. Human CCND3 contains a PEST domain at amino acids 256-268 (inclusive) and 271-291 (inclusive). In investigating the role of PEST domains, researchers have found that a single allele SNP in CCND3 resulted in an amino acid change from serine to alanine at amino acid 259 (S259A). The S259A change disrupted the PEST domain and as a result, the variant CCND3 had increased stability when compared to the non-mutated allele (Savas et al., OMICS 2007, 11(2): 200-208).

CCND3 is required in the development of lymphocytes. When CCND3 knockout mice (CCND3 -/-) were generated in order to test CCND3 function (Sicinska et al., Cancer Cell 2003, 4(6):451-461), these knockout mice did not undergo normal expansion of immature T cells, a very tissue specific defect. To address the role that CCND3 can play in T-cell malignancies, the CCND3 knockout mice were crossed with mice overexpressing p56LCK, which display high numbers of T-cell tumors. The mice expressing wild type CCND3/p56LCK formed T-cell malignancies quickly. In contrast, the formation of T-cell malignancies was delayed by 6-8 weeks in CCND3 knockout/p56LCK mice. Using this data to investigate human malignancies, the same group used an siRNA approach to knockdown CCND3 in human T-cell acute lymphoblastic leukemia (T-ALL) cell lines. Knockdown of CCND3 in all 12 cell lines tested significantly inhibited cell proliferation, confirming the mouse data and demonstrating the importance of CCND3 in human T-cell malignancies.

Improvements in the ease, speed and cost of DNA sequencing technologies has allowed investigators to examine the whole genome of a cancer cell for alterations. Taking this approach, two groups performed a whole genome analysis of diffuse large B-cell lymphoma (DLBCL) in order to find specific genes and pathways which may be responsible for this cancer (Pasqualucci et al., Nature Genetics 2011, 43(9): 830-837: Morin et al., Nature 2011, 476:298-303). This approach found CCND3 mutations in 3/54 of the DLBCLs tested. WO-2009/150491 relates to a method of evaluating whether a cyclin-dependent kinase (CDK) inhibitor will inhibit growth of a cancer cell. The method comprises measuring the amount of IFNEI mRNA or protein in the cancer cell before administering the CDK inhibitor.

In this disclosure, a CCND3 mutation is used as a biomarker or indicator, predicting and targeting patients for a specific therapy. Finding biomarkers which indicate which patient should receive a therapeutic is useful, especially with regard to cancer. This allows for more timely and aggressive treatment as opposed to a trial and error approach. In addition, the discovery of biomarkers which indicate that cells continue to be sensitive to the therapy after administration is also useful. These biomarkers can be used to monitor the response of those patients receiving the therapeutic. If biomarkers indicate that the patient has become insensitive to the treatment, then the dosage administered can be increased, decreased, completely discontinued or an additional therapeutic administered. As such, there is a need to develop biomarkers associated with Cyclin Dependant Kinase (CDK) inhibitors. This approach ensures that the correct patients receive the appropriate treatment and during the course of the treatment the patient can be monitored for continued CDK inhibitor sensitivity.

In the development of CDK inhibitors, specific biomarkers will aid in understanding the mechanism of action upon administration. The mechanism of action may involve a complex cascade of regulatory mechanisms in the cell cycle and differential gene expression. This analysis is done at the pre-clinical stage of drug development in order to determine the particular sensitivity of cancer cells to the CDK inhibitor candidate and the activity of the candidate. Of particular interest in the pharmacodynamic investigation is the identification of specific markers of sensitivity and activity, such as the ones disclosed herein.

### SUMMARY OF THE INVENTION

The disclosure provides that mutation in the Cyclin D3 (henceforth "CCND3") PEST domain act as a specific biomarker in determining the sensitivity of cells to inhibitors of Cyclin Dependant Kinase 4 and/or Cyclin Dependant Kinase 6 (henceforth "CDKi"). The disclosure includes a CCND3 mutational analysis that results in a "gene signature" for CDKi that has increased accuracy and specificity in predicting which cancer cells are sensitive to a CDKi. The method analyzes CCND3 mutation in a PEST domain, in a cancer sample taken from a patient and compared to a non-cancerous or normal control and predicts the sensitivity of the cancer sample to a CDKi. The analysis of CCND3 mutation can be indicative of a favorable response or an unfavorable one. The invention is an example of "personalized medicine" wherein patients are treated based on a functional genomic signature that is specific to that individual.

The predictive value of CCND3 mutations can also be used after treatment with a CDKi to determine if the patient remains sensitive to the treatment. Once the CDKi therapeutic has been administered, a CCND3 mutation can be used as a biomarker to monitor the continued sensitivity of the patient to CDKi treatment. This is useful in determining that patients receive the correct course of treatment. The disclosure comprises a method of predicting and monitoring the sensitivity of a patient to CDKi treatment. The method includes the step of administration of a CDKi to a patient and analyzing for a CCND3 mutation on a biological sample obtained from the treated patient and comparing it CCND3 in a non-cancerous or normal sample. The response of the patient is evaluated based on the detection of a CCND3 mutation in a PEST domain. In addition, detection and/or alteration in the level of CCND3 protein expression from a cell containing a CCND3 mutation compared to a normal or wild-type control cell can be predictive of the sensitivity of the patient to the treatment. The pattern of CCND3 mutant protein expression level changes can be indicative of a favorable patient response or an unfavorable one.

### DESCRIPTION OF THE DRAWINGS

Figure 1 is a graphic of the CCND3 protein and certain mutations. The PEST domains of CCND3 are amino acids 256-268 and 271-291 of human CCND3.
Figure 2A is a graph of cell viability for cells containing wild type CCND3 that demonstrate insensitivity to CDKi(1).
Figure 2B is a graph of cell viability for cells containing mutant CCND3 that demonstrate sensitivity to CDKi(1).
Figure 2C is a box plot of EC⁵⁰ values for wild type and mutated CCND3 cell lines treated with CDKi(1).
Figure 3A is a graph of cell viability for cells containing wild type CCND3 that demonstrate insensitivity to CDKi(2).
Figure 3B is a graph of cell viability for cells containing mutant CCND3 that demonstrate sensitivity to CDKi(2).
Figure 3C is a box plot of EC50 values for wild type and mutated CCND3 cell lines treated with CDKi(2).
Figure 4A is a graph of cell viability for cells containing wild type CCND3 that demonstrate insensitivity to CDKi(3).
Figure 4B is a graph of cell viability for cells containing mutant CCND3 that demonstrate insensitivity to CDKi(3).
Figure 4C is a box plot of EC50 values for wild type and mutated CCND3 cell lines treated with CDKi(3).
Figures 5A/B are Western Blots demonstrating that CCND3 mutations result in increased protein stability.
Figure 6A is a Western Blot showing that cells containing a CCND3 mutation result in higher levels of CCND3 mutant protein.
Figure 6B is a graphic representation of mRNA expression in wild type cell lines and cell lines containing a CCND3 mutation.

### DESCRIPTION OF THE INVENTION

The invention is defined by the appended claims. Disclosed herein is a method of determining the sensitivity of a cancer cell to a Cyclin Dependant Kinase inhbitor (CDKi), the method comprising: a) assaying for a cyclin D3 (CCND3) mutation in a cancer cell; and b) comparing the CCND3 mutation with a non-cancerous or normal control cell wherein the presence of a CCND3 mutation in the cancer cell indicates it is sensitive to a CDKi.

The method wherein the cancer cell is selected from the group consisting of: diffuse large B cell lymphoma, lymphoma, lymphocytic leukemia, acute lymphoblastic B cell leukemia and Burkitts lymphoma.

The method wherein the CCND3 mutation is in a PEST domain.

The method, wherein the CCND3 mutation is at least one amino acid change in amino acids 256-268 of SEQ ID NO. 2

The method wherein the CCND3 mutation is at least one amino acid change in amino acids 271-292 of SEQ ID NO. 2

The method wherein the CCND3 mutation is any mutation in Table 2.

The method wherein the CCND3 mutation is selected from the group consisting of: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D).

The method wherein the CDKi is selected from Table 1.

Disclosed herein is a method of predicting the sensitivity of a cancer patient for treatment with a CDKi, the method comprising: a) assaying for a CCND3 mutation in a cancer sample obtained from the patient; and b) comparing the CCND3 mutation with a non-cancerous or normal control sample wherein the presence of a CCND3 mutation in the cancer sample indicates that the patient is sensitive to treatment with a CDKi.

The method wherein the cancer sample is selected from the group consisting of: diffuse large B cell lymphoma, lymphoma, lymphocytic leukemia, acute lymphoblastic B cell leukemia and Burkitts lymphoma.

The method wherein the CCND3 mutation is in a PEST domain.

The method wherein the CCND3 mutation is at least one amino acid change in amino acids 256-268 of SEQ ID NO. 2

The method wherein the CCND3 mutation is at least one amino acid change in amino acids 271-292 of SEQ ID NO. 2

The method wherein the CCND3 mutation is any mutation in Table 2.

The method wherein the CCND3 mutation is selected from the group consisting of: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D).

The method wherein the CDKi is selected from Table 1.

The method further comprising: c) measuring differential protein expression of CCND3 in a cancer sample obtained from the patient; and d) comparing the protein expression of CCND3 in the cancer sample with CCND3 protein expression of a non-cancerous or normal control sample, wherein increased levels of CCND3 protein in the cancer sample indicate that the patient is sensitive to treatment with a CDKi.

Disclosed herein is a method of treating a cancer patient with a CDKi, the method comprising: a) assaying for CCND3 mutations in a cancer sample obtained from the patient; b) comparing the CCND3 mutational status in the cancer sample with a non-cancerous or normal control sample wherein the presence of a CCND3 mutation indicates that the patient is sensitive to treatment with a CDKi; c) administering to the patient a CDKi; and d) assaying for suppression of tumor growth.

The method wherein the cancer sample is selected from the group consisting of: diffuse large B cell lymphoma, lymphoma, lymphocytic leukemia, acute lymphoblastic B cell leukemia and Burkitts lymphoma.

The method wherein the CCND3 mutation is in a PEST domain.

The method wherein the CCND3 mutation is at least one amino acid change in amino acids 256-268 of SEQ ID NO. 2

The method wherein the CCND3 mutation is at least one amino acid change in amino acids 271-292 of SEQ ID NO. 2

The method wherein the CCND3 mutation is any mutation in Table 2.

The method wherein the CCND3 mutation is selected from the group consisting of: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D).

The method wherein the CDKi is administered in a therapeutically effective amount.

The method wherein the CDKi is selected from Table 1.

Disclosed herein is a method of screening for CDKi candidates the method comprising: a) contacting a cell containing a CCND3 mutation with a CDKi candidate; b) measuring the reduction in cell viability; and c) comparing the reduction in cell viability from the CCND3 mutant cell contacted with the CDKi candidate with cell viability of the CCND3 mutant cell contacted with a control CDKi.

The method wherein the control CDKi is selected from Table 1.

The method wherein the cell containing a CCND3 mutation is selected from the group consisting of diffuse large B cell lymphoma, lymphoma, lymphocytic leukemia, acute lymphoblastic B cell leukemia and Burkitts lymphoma.

The method wherein the CCND3 mutation is in a PEST domain.

The method wherein the CCND3 mutation is at least one amino acid change in amino acids 256-268 of SEQ ID NO. 2

The method wherein the CCND3 mutation is at least one amino acid change in amino acids 271-292 of SEQ ID NO. 2

The method wherein the CCND3 mutation is any mutation in Table 2.

The method wherein the CCND3 mutation is selected from the group consisting of: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D).

The invention relates to a composition comprising a Cyclin Dependent Kinase inhibitor (CDKi) for use in treatment of cancer in a selected cancer patient population, wherein the cancer patient population is selected on the basis of showing a CCND3 mutation in a cancer cell sample obtained from said patient compared to a normal control cell sample, wherein the CCND3 mutation is in a PEST domain, wherein the cancer sample is selected from the group consisting of diffuse large B cell lymphoma, lymphoma, lymphocytic leukemia, acute lymphoblastic B cell leukemia and Burkitts lymphoma.

Disclosed herein is a kit for predicting the sensitivity of a cancer patient for treatment with a CDKi comprising:i) means for detecting CCND3 mutation; and ii) instructions how to use said kit.

### Definitions

As used in the specification and claims, the singular form "a", "an" and "the" include plural references unless the context clearly dictates otherwise. For example, the term "a cell" includes a plurality of cells, including mixtures thereof.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about." It also is to be understood, although not always explicitly stated, that the reagents described herein are merely exemplary and that equivalents of such are known in the art.

The terms "marker" or "biomarker" are used interchangeably herein. A biomarker is a nucleic acid or polypeptide and the presence or absence of a mutation or differential expression of the polypeptide is used to determine sensitivity to any CDKi. For example, CCND3 is a biomarker in a cancer cell when it is mutated as compared to CCND3 in normal (non-cancerous) cell or control cell.

A cell is "sensitive" or displays "sensitivity" for inhibition with a CDKi when the cell viability is reduced upon treatment with the CDKi when compared to an untreated control.

"CCND3" refers to the Cyclin D3 gene. Unless specifically stated otherwise, CCND3 as used herein, refers to human CCND3, accession numbers BC011616 (CCND3 nucleic acid (SEQ ID NO. 1)) and AAH11616 (CCND3 protein (SEQ ID NO.2)).

A "wild-type," "normal," or "non-mutant" refers to sequences of CCND3 comprising accession numbers BC011616/ AAH11616 ((nucleic acid (SEQ ID NO. 1) and protein (SEQ ID NO.2) respectively)).

A "mutant," or "mutation" is any change in DNA or protein sequence that deviates from wild type CCND3. This includes without limitation; single base nucleic acid changes or single amino acid changes, insertions, deletions and truncations of the CCND3 gene and its corresponding protein.

A "control cell," "normal cell" or "wild-type" refers to non-cancerous tissue or cells.

A "control tissue," "normal tissue" or "wild-type" refers to non-cancerous tissue or cells.

The terms "nucleic acid" and "polynucleotide" are used interchangeably and refer to a polymeric form of nucleotides of any length, either deoxyribonucleotides or ribonucleotides or analogs thereof. Polynucleotides can have any three-dimensional structure and can perform any function. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide can comprise modified nucleotides, such as methylated nucleotides and nucleotide analogs. If present, modifications to the nucleotide structure can be imparted before or after assembly of the polymer. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

A "gene" refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

"Gene expression" or alternatively a "gene product" refers to the nucleic acids or amino acids (e.g., peptide or polypeptide) generated when a gene is transcribed and translated.

The term "polypeptide" is used interchangeably with the term "protein" and in its broadest sense refers to a compound of two or more subunit amino acids, amino acid analogs, or peptidomimetics. The subunits can be linked by peptide bonds. In another embodiment, the subunit may be linked by other bonds, e.g., ester, ether, etc.

As used herein the term "amino acid" refers to either natural and/or unnatural or synthetic amino acids, and both the D and L optical isomers, amino acid analogs, and peptidomimetics. A peptide of three or more amino acids is commonly called an oligopeptide if the peptide chain is short. If the peptide chain is long, the peptide is commonly called a polypeptide or a protein.

The term "isolated" means separated from constituents, cellular and otherwise, in which the polynucleotide, peptide, polypeptide, protein, antibody or fragment(s) thereof, are normally associated with in nature. For example, an isolated polynucleotide is separated from the 3' and 5' contiguous nucleotides with which it is normally associated within its native or natural environment, e.g., on the chromosome. As is apparent to those of skill in the art, a non-naturally occurring polynucleotide, peptide, polypeptide, protein, antibody, or fragment(s) thereof, does not require "isolation" to distinguish it from its naturally occurring counterpart. In addition, a "concentrated," "separated" or "diluted" polynucleotide, peptide, polypeptide, protein, antibody or fragment(s) thereof, is distinguishable from its naturally occurring counterpart in that the concentration or number of molecules per volume is greater in a "concentrated" version or less than in a "separated" version than that of its naturally occurring counterpart. A polynucleotide, peptide, polypeptide, protein, antibody, or fragment(s) thereof, which differs from the naturally occurring counterpart in its primary sequence or, for example, by its glycosylation pattern, need not be present in its isolated form since it is distinguishable from its naturally occurring counterpart by its primary sequence or, alternatively, by another characteristic such as glycosylation pattern. Thus, a non-naturally occurring polynucleotide is provided as a separate embodiment from the isolated naturally occurring polynucleotide. A protein produced in a bacterial cell is provided as a separate embodiment from the naturally occurring protein isolated from a eukaryotic cell in which it is produced in nature.

A "probe" when used in the context of polynucleotide manipulation refers to an oligonucleotide that is provided as a reagent to detect a target potentially present in a sample of interest by hybridizing with the target. Usually, a probe will comprise a label or a means by which a label can be attached, either before or subsequent to the hybridization reaction. Suitable labels include, but are not limited to radioisotopes, fluorochromes, chemiluminescent compounds, dyes, and proteins, including enzymes.

A "primer" is a short polynucleotide, generally with a free 3'-OH group that binds to a target or "template" potentially present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or a "set of primers" consisting of an "upstream" and a "downstream" primer, and a catalyst of polymerization, such as a DNA polymerase, and typically a thermally-stable polymerase enzyme. Methods for PCR are well known in the art, and taught, for example in PCR: A Practical Approach, M. MacPherson et al., IRL Press at Oxford University Press (1991). All processes of producing replicate copies of a polynucleotide, such as PCR or gene cloning, are collectively referred to herein as "replication." A primer can also be used as a probe in hybridization reactions, such as Southern or Northern blot analyses (Sambrook et al., Molecular Cloning: A Laboratory Manual, 2nd edition (1989)).

As used herein, "expression" refers to the process by which DNA is transcribed into mRNA and/or the process by which the transcribed mRNA is subsequently translated into peptides, polypeptides or proteins. If the polynucleotide is derived from genomic DNA, expression may include splicing of the mRNA in a eukaryotic cell.

"Differentially expressed" as applied to a gene, refers to the differential production of the mRNA transcribed and/or translated from the gene or the protein product encoded by the gene. A differentially expressed gene may be overexpressed or underexpressed as compared to the expression level of a normal or control cell. However, as used herein, overexpression is an increase in gene expression and generally is at least 1.25 fold or, alternatively, at least 1.5 fold or, alternatively, at least 2 fold, or alternatively, at least 3 fold or alternatively, at least 4 fold expression over that detected in a normal or control counterpart cell or tissue. As used herein, underexpression, is a reduction of gene expression and generally is at least 1.25 fold, or alternatively, at least 1.5 fold, or alternatively, at least 2 fold or alternatively, at least 3 fold or alternatively, at least 4 fold expression under that detected in a normal or control counterpart cell or tissue. The term "differentially expressed" also refers to where expression in a cancer cell or cancerous tissue is detected but expression in a control cell or normal tissue (e.g. non cancerous cell or tissue) is undetectable.

A high expression level of the gene can occur because of over expression of the gene or an increase in gene copy number. The gene can also be translated into increased protein levels because of deregulation or absence of a negative regulator. Lastly, high expression of the gene can occur due to increased stabilization or reduced degradation of the protein, resulting in accumulation of the protein.

A "gene expression profile" or "gene signature" refers to a pattern of expression of at least one biomarker that recurs in multiple samples and reflects a property shared by those samples, such as mutation, response to a particular treatment, or activation of a particular biological process or pathway in the cells. A gene expression profile differentiates between samples that share that common property and those that do not with better accuracy than would likely be achieved by assigning the samples to the two groups at random. A gene expression profile may be used to predict whether samples of unknown status share that common property or not. Some variation between the biomarker(s) and the typical profile is to be expected, but the overall similarity of biomarker(s) to the typical profile is such that it is statistically unlikely that the similarity would be observed by chance in samples not sharing the common property that the biomarker(s) reflects.

The term "cDNA" refers to complementary DNA, i.e. mRNA molecules present in a cell or organism made into cDNA with an enzyme such as reverse transcriptase. A "cDNA library" is a collection of all of the mRNA molecules present in a cell or organism, all turned into cDNA molecules with the enzyme reverse transcriptase, then inserted into "vectors" (other DNA molecules that can continue to replicate after addition of foreign DNA). Exemplary vectors for libraries include bacteriophage (also known as "phage"), viruses that infect bacteria, for example, lambda phage. The library can then be probed for the specific cDNA (and thus mRNA) of interest.

As used herein, "solid phase support" or "solid support", used interchangeably, is not limited to a specific type of support. Rather a large number of supports are available and are known to one of ordinary skill in the art. Solid phase supports include silica gels, resins, derivatized plastic films, glass beads, plastic beads, alumina gels, microarrays, and chips. As used herein, "solid support" also includes synthetic antigen-presenting matrices, cells, and liposomes. A suitable solid phase support may be selected on the basis of desired end use and suitability for various protocols. For example, for peptide synthesis, solid phase support may refer to resins such as polystyrene (e.g., PAM-resin obtained from Bachem Inc., Peninsula Laboratories), polyHIPE(R)™ resin (obtained from Aminotech, Canada), polyamide resin (obtained from Peninsula Laboratories), polystyrene resin grafted with polyethylene glycol (TentaGeIR™, Rapp Polymere, Tubingen, Germany), or polydimethylacrylamide resin (obtained from Milligen/Biosearch, California).

A polynucleotide also can be attached to a solid support for use in high throughput screening assays. PCT WO 97/10365, for example, discloses the construction of high density oligonucleotide chips. See also, U.S. Pat. Nos. 5,405,783; 5,412,087 and 5,445,934. Using this method, the probes are synthesized on a derivatized glass surface to form chip arrays. Photoprotected nucleoside phosphoramidites are coupled to the glass surface, selectively deprotected by photolysis through a photolithographic mask and reacted with a second protected nucleoside phosphoramidite. The coupling/deprotection process is repeated until the desired probe is complete.

As an example, transcriptional activity can be assessed by measuring levels of messenger RNA using a gene chip such as the Affymetrix® HG-U133-Plus-2 GeneChips (Affymetrix, Santa Clara, CA). High-throughput, real-time quanititation of RNA of a large number of genes of interest thus becomes possible in a reproducible system.

The terms "stringent hybridization conditions" refers to conditions under which a nucleic acid probe will specifically hybridize to its target subsequence, and to no other sequences. The conditions determining the stringency of hybridization include: temperature, ionic strength, and the concentration of denaturing agents such as formamide. Varying one of these factors may influence another factor and one of skill in the art will appreciate changes in the conditions to maintain the desired level of stringency. An example of a highly stringent hybridization is: 0.015M sodium chloride, 0.0015M sodium citrate at 65-68 °C or 0.015M sodium chloride, 0.0015M sodium citrate, and 50% formamide at 42 °C (see Sambrook, *supra*). An example of a "moderately stringent" hybridization is the conditions of: 0.015M sodium chloride, 0.0015M sodium citrate at 50-65 °C or 0.015M sodium chloride, 0.0015M sodium citrate, and 20% formamide at 37-50 °C. The moderately stringent conditions are used when a moderate amount of nucleic acid mismatch is desired. One of skill in the art will appreciate that washing is part of the hybridization conditions. For example, washing conditions can include 02.X-0.1X SSC/0.1% SDS and temperatures from 42-68 °C, wherein increasing temperature increases the stringency of the wash conditions.

When hybridization occurs in an antiparallel configuration between two single-stranded polynucleotides, the reaction is called "annealing" and those polynucleotides are described as "complementary." A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to form hydrogen bonding with each other, according to generally accepted base-pairing rules.

A polynucleotide or polynucleotide region (or a polypeptide or polypeptide region) has a certain percentage (for example, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art, for example those described in Current Protocols in Molecular Biology, Ausubel et al., eds., (1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code=standard; filter=none; strand=both; cutoff=60; expect=10; Matrix=BLOSUM62; Descriptions=50 sequences; sort by=HIGH SCORE; Databases=non-redundant.

The term "cell proliferative disorders" shall include dysregulation of normal physiological function characterized by abnormal cell growth and/or division or loss of function. Examples of "cell proliferative disorders" includes but is not limited to hyperplasia, neoplasia, metaplasia, and various autoimmune disorders, e.g., those characterized by the dysregulation of T cell apoptosis.

As used herein, the terms "neoplastic cells," "neoplastic disease," "neoplasia," "tumor," "tumor cells," "cancer," and "cancer cells," (used interchangeably) refer to cells which exhibit relatively autonomous growth, so that they exhibit an aberrant growth phenotype characterized by a significant loss of control of cell proliferation (i.e., de-regulated cell division). Neoplastic cells can be malignant or benign. A "metastatic cell or tissue" means that the cell can invade and destroy neighboring body structures. Cancer can include without limitation, diffuse Large B cell lymphoma, lymphoma, lymphocytic leukemia, acute lymphoblastic B cell leukemia and Burkitts lymphoma.

The term "PBMC" refers to peripheral blood mononuclear cells and includes "PBL" - peripheral blood lymphocytes.

"Suppressing" or "suppression" of tumor growth indicates a reduction in tumor cell growth when contacted with a CDKi compared to tumor growth without contact with a CDKi compound. Tumor cell growth can be assessed by any means known in the art, including, but not limited to, measuring tumor size, determining whether tumor cells are proliferating using a 3H-thymidine incorporation assay, measuring glucose uptake by FDG-PET (fluorodeoxyglucose positron emission tomography) imaging, or counting tumor cells. "Suppressing" tumor cell growth means any or all of the following states: slowing, delaying and stopping tumor growth, as well as tumor shrinkage.

A "composition" is a combination of active agent and another carrier, e.g., compound or composition, inert (for example, a detectable agent or label) or active, such as an adjuvant, diluent, binder, stabilizer, buffers, salts, lipophilic solvents, preservative, adjuvant or the like. Carriers also include pharmaceutical excipients and additives, for example; proteins, peptides, amino acids, lipids, and carbohydrates (e.g., sugars, including monosaccharides and oligosaccharides; derivatized sugars such as alditols, aldonic acids, esterified sugars and the like; and polysaccharides or sugar polymers), which can be present singly or in combination, comprising alone or in combination 1-99.99% by weight or volume. Carbohydrate excipients include, for example; monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

Exemplary protein excipients include serum albumin such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like.

The term "carrier" further includes a buffer or a pH adjusting agent; typically, the buffer is a salt prepared from an organic acid or base. Representative buffers include organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. Additional carriers include polymeric excipients/additives such as polyvinylpyrrolidones, ficolls (a polymeric sugar), dextrates (e.g., cyclodextrins, such as 2-hydroxypropyl-quadrature-cyclodextrin), polyethylene glycols, flavoring agents, antimicrobial agents, sweeteners, antioxidants, antistatic agents, surfactants (e.g., polysorbates such as TWEEN 20™ and TWEEN 80™), lipids (e.g., phospholipids, fatty acids), steroids (e.g., cholesterol), and chelating agents (e.g., EDTA).

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives and any of the above noted carriers with the additional provisio that they be acceptable for use in vivo. For examples of carriers, stabilizers and adjuvants, see Remington's Pharmaceutical Science., 15th Ed. (Mack Publ. Co., Easton (1975) and in the Physician's Desk Reference, 52nd ed., Medical Economics, Montvale, N.J. (1998).

An "effective amount" is an amount sufficient to effect beneficial or desired results. An effective amount can be administered in one or more administrations, applications or dosages.

A "subject," "individual" or "patient" is used interchangeably herein, which refers to a vertebrate, preferably a mammal, more preferably a human. Mammals include, but are not limited to, mice, simians, humans, farm animals, sport animals, and pets.

An "inhibitor" of CDK (CDKi) as used herein reduces the association of CCND3 and CDK4 and/or CDK6. This inhibition may include, for example, reducing the association of CCND3 and CDK4/6 before they are bound together, or reducing the association of CCND3 and CDK4/6 after they are bound together, thus freeing both molecules. The reduction can range from a low, but detectable amount to complete disassociation of the molecules.

A number of CCND3 mutations have now been identified as biomarkers for CDKi. A CCND3 mutation in a PEST domain can be used to determine patient sensitivity to any CDKi. CCND3 mutations include without limitation, insertions, deletions, frameshifts, and one or more point mutations in a PEST domain. For example and without limitation, CCND3 mutation in the PEST domain include: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D). For example, a mutation in CCND3 in a PEST domain at amino acid 290 from isoleucine to threonine (I290T), indicates that a cancer patient is sensitive to and would favorably respond to administration of any CDKi.

CDK inhibitors (CDKi) are compounds that are inhibitors of the CCND3 - CDK4/6 association, and are useful in the compositions for use of the invention. CDKi are useful in pharmaceutical compositions for human or veterinary use where inhibition of the CCND3 - CDK4/6 association is indicated, e.g., in the treatment of tumors and/or cancerous cell growth. CDKi compounds are useful in treating, for example, diffuse large B cell lymphoma, lymphoma, lymphocytic leukemia, acute lymphoblastic B cell leukemia and Burkitts lymphoma.

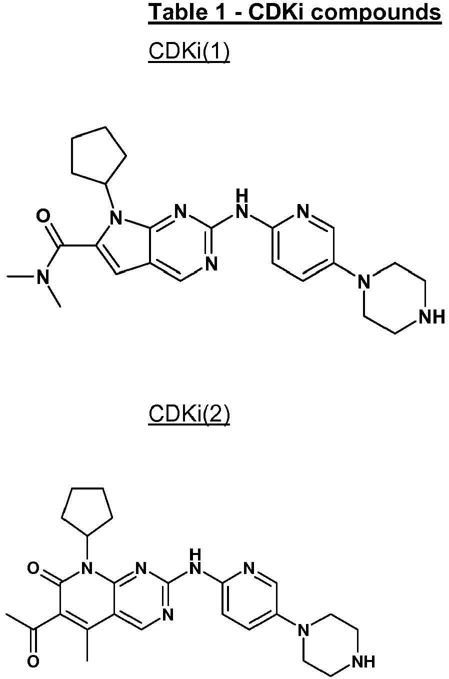

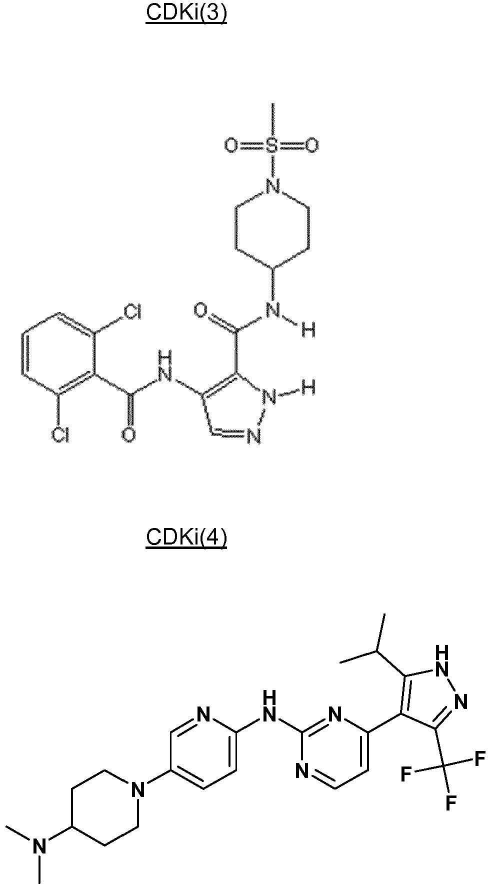

**Table 2**

| **Hugo Symbol** | **Cell Line** | **cDNA Change** | **Protein Change** | **UniProt AApos** | **iMutant allele Frequency** | **PolyPhen Prediction** | **Simple Prediction** | **Histologic Subtype** |
|---|---|---|---|---|---|---|---|---|
| CCND3 | a4fuk | c.869T>A | p.I290K | 290 | 0.509091 | Benign | MILD | Diffuse Large B Cell Lymphoma |
| CCND3 | bI70 | c.801_802insC | p.P267fs | 267_268 | 0.52 | | | Lymphoma, B cell, Non-Hodgkins, Burkitts |
| CCND3 | dohh2 | c.869T>C | p.I290T | 290 | 0.45283 | Benign | MILD | Diffuse Large B Cell Lymphoma |
| CCND3 | mc116 | c.868_874delATACACC | p.I290fs | 290_292 | 0.38 | | | Lymphoma B Cell, Non Hodgkins, Unspecified |
| CCND3 | mec1 | c.860T>A | p.V287D | 287 | 0.692308 | Probably Damaging | Damaging Missense | Chronic Lymphocytic Leukemia |
| CCND3 | nudhl1 | c.850C>T | p.P284S | 284 | 0.589189 | Probably Damaging | Damaging Missense | Diffuse Large B Cell Lymphoma |
| CCND3 | sem | c.869T>C | p.I290T | 290 | 0,520661 | Benign | MILD | Acute lymphoblastic B cell leukemia |
| CCND3 | st486 | c.851C>T | p.P284L | 284 | 0.39881 | Probably Damaging | Neutral Missense | Burkitts Lymphoma |
| CCND3 | sudhl10 | c.869T>C | p.I290T | 290 | 0.439024 | Benign | MILD | Diffuse Large B Cell Lymphoma |

### Detection of CCND3 mutations

The detection of CCND3 mutations can be done by any number of ways, for example: DNA sequencing, PCR based methods, including RT-PCR, microarray analysis, Southern blotting, Northern blotting and dip stick analysis.

The polymerase chain reaction (PCR) can be used to amplify and identify CCND3 mutations from either genomic DNA or RNA extracted from tumor tissue. PCR is well known in the art and is described in detail in Saiki et al., Science 1988, 239:487 and in U.S. Patent No. 4,683,195 and U.S. Patent No. 4,683,203.

Methods of detecting CCND3 mutations by hybridization are provided. The method comprises identifying a CCND3 mutation in a sample by contacting nucleic acid from the sample with a nucleic acid probe that is capable of hybridizing to nucleic acid with a CCND3 mutation or fragment thereof and detecting the hybridization. The nucleic acid probe is detectably labeled with a label such as a radioisotope, a fluorescent agent or a chromogenic agent. Radioisotopes can include without limitation; 3^{H}, 32^{P}, 33^{P} and 35^{S} etc. Fluorescent agents can include without limitation: FITC, texas red, rhodamine, etc.

The probe used in detection that is capable of hybridizing to nucleic acid with a CCND3 mutation can be from about 8 nucleotides to about 100 nucleotides, from about 10 nucleotides to about 75 nucleotides, from about 15 nucleotides to about 50 nucleotides, or about 20 to about 30 nucleotides. The probe or probes can be provided in a kit, which comprise at least one oligonucleotide probe that hybridizes to or hybridizes adjacent to a CCND3 mutation. The kit can also provide instructions for analysis of patient cancer samples that can contain a CCND3 mutation, and which CCND3 mutations indicate that the patient is sensitive or insensitive to treatment with a CDKi.

Single stranded conformational polymorphism (SSCP) can also be used to detect CCND3 mutations. This technique is well described in Orita et al., PNAS 1989, 86:2766-2770.

Antibodies directed against CCND3 can be useful in the detection of cancer and the detection of mutated forms of CCND3. Antibodies can be generated which recognize and specifically bind only a specific mutant of CCND3 and do not bind (or weakly bind) to wild type CCND3. These antibodies would be useful in determining which specific mutation was present and also in quantifying the level of CCND3 protein. For example, an antibody can be directed against the isoleucine to threonine change at amino acid position 290 (I290T). An antibody that recognizes this amino acid change and does not specifically bind to wild type CCND3 could identify the specific mutation in tissue sections and also the protein levels by Western blotting. Such antibodies can be generated against a CCND3 mutation by using peptides containing the specific CCND3 mutation of interest.

Antibodies than can distinguish between phosphorylated and non-phosphorylated epitopes are known in the art (Luca et al., PNAS USA 1986 83(4):1006-1010). Antibodies directed against the non-phosphorlyated form of CCND3 at the threonine at position 283(T283) in the PEST domain can be also be useful in the detection of mutant forms of CCND3. For example, a mutation in a PEST domain of CCND3 can reduce or prevent the phosphorlyation of T283. A staining procedure with an antibody that recognizes only the non-phosphorylated form of CCND3 combined with an antibody that recognizes only a mutant form of CCND3 would further validate and confirm that the patient sample is sensitive to a CDKi. In another example, PCR could be performed on a cancer sample to detect the CCND3 mutation, using standard PCR techniques as described above. If a CCND3 mutation is indicated by PCR, the protein of the cancer sample can be analyzed by an anti-CCND3 antibody that is directed to the phosphorylated epitope. A positive result from the PCR reaction and positive staining from the antibody indicating that CCND3 is not phosphorylated, would determine that the patient would be sensitive to treatment with a CDKi.

A cancer cell believed to contain a CCND3 mutation can be lysed and Western blotting performed to quantitate the amount of CCND3 mutant protein, using a cell containing wild type CCND3 as a control. Little or no detection of the phosphorylated form of CCND3, combined with detection of higher protein levels in the cancer cell when compared with wild-type CCND3 in a normal cell would indicate that a mutation has occurred in the CCND3 in cancer cell that reduces or prevents phosphorlyation and thus this cancer cell would be sensitive to a CDKi.

### Measurement of Gene Expression

Detection of gene expression can be by any appropriate method, including for example, detecting the quantity of mRNA transcribed from the gene or the quantity of cDNA produced from the reverse transcription of the mRNA transcribed from the gene or the quantity of the polypeptide or protein encoded by the gene. These methods can be performed on a sample by sample basis or modified for high throughput analysis. For example, using Affymetrix™ U133 microarray chips.

In one aspect, gene expression is detected and quantitated by hybridization to a probe that specifically hybridizes to the appropriate probe for that biomarker. The probes also can be attached to a solid support for use in high throughput screening assays using methods known in the art. WO 97/10365 and U.S. Pat. Nos. 5,405,783, 5,412,087 and 5,445,934, for example, disclose the construction of high density oligonucleotide chips which can contain one or more of the sequences disclosed herein. Using the methods disclosed in U.S. Pat. Nos. 5,405,783, 5,412,087 and 5,445,934, the probes of this invention are synthesized on a derivatized glass surface. Photoprotected nucleoside phosphoramidites are coupled to the glass surface, selectively deprotected by photolysis through a photolithographic mask, and reacted with a second protected nucleoside phosphoramidite. The coupling/deprotection process is repeated until the desired probe is complete.

In one aspect, the expression level of a gene is determined through exposure of a nucleic acid sample to the probe-modified chip. Extracted nucleic acid is labeled, for example, with a fluorescent tag, preferably during an amplification step. Hybridization of the labeled sample is performed at an appropriate stringency level. The degree of probe-nucleic acid hybridization is quantitatively measured using a detection device. See U.S. Pat. Nos. 5,578,832 and 5,631,734.

Alternatively any one of gene copy number, transcription, or translation can be determined using known techniques. For example, an amplification method such as PCR may be useful. General procedures for PCR are taught in MacPherson et al., PCR: A Practical Approach, (IRL Press at Oxford University Press (1991)). However, PCR conditions used for each application reaction are empirically determined. A number of parameters influence the success of a reaction. Among them are annealing temperature and time, extension time, Mg 2+ and /or ATP concentration, pH, and the relative concentration of primers, templates, and deoxyribonucleotides. After amplification, the resulting DNA fragments can be detected by agarose gel electrophoresis followed by visualization with ethidium bromide staining and ultraviolet illumination.

In one embodiment, the hybridized nucleic acids are detected by detecting one or more labels attached to the sample nucleic acids. The labels can be incorporated by any of a number of means well known to those of skill in the art. However, in one aspect, the label is simultaneously incorporated during the amplification step in the preparation of the sample nucleic acid. Thus, for example, polymerase chain reaction (PCR) with labeled primers or labeled nucleotides will provide a labeled amplification product. In a separate embodiment, transcription amplification, as described above, using a labeled nucleotide (e.g. fluorescein-labeled UTP and/or CTP) incorporates a label in to the transcribed nucleic acids.

Alternatively, a label may be added directly to the original nucleic acid sample (e.g., mRNA, polyA, mRNA, cDNA, etc.) or to the amplification product after the amplification is completed. Means of attaching labels to nucleic acids are well known to those of skill in the art and include, for example nick translation or end-labeling (e.g. with a labeled RNA) by kinasing of the nucleic acid and subsequent attachment (ligation) of a nucleic acid linker joining the sample nucleic acid to a label (e.g., a fluorophore).

Detectable labels suitable for use in the present disclosure include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, texas red, rhodamine, green fluorescent protein, and the like), radiolabels (e.g., 3^{H}, 125^{I}, 35^{S}, 14^{C}, or 32^{P}) enzymes (e.g., horse radish peroxidase, alkaline phosphatase and others commonly used in an ELISA), and calorimetric labels such as colloidal gold or colored glass or plastic (e.g., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241.

Detection of labels is well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and calorimetric labels are detected by simply visualizing the coloured label.

The detectable label may be added to the target (sample) nucleic acid(s) prior to, or after the hybridization, such as described in WO 97/10365. These detectable labels are directly attached to or incorporated into the target (sample) nucleic acid prior to hybridization. In contrast, "indirect labels" are joined to the hybrid duplex after hybridization. Generally, the indirect label is attached to a binding moiety that has been attached to the target nucleic acid prior to the hybridization. For example, the target nucleic acid may be biotinylated before the hybridization. After hybridization, an avidin-conjugated fluorophore will bind the biotin bearing hybrid duplexes providing a label that is easily detected. For a detailed review of methods of labeling nucleic acids and detecting labeled hybridized nucleic acids see Laboratory Techniques in Biochemistry and Molecular Biology, Vol. 24: Hybridization with Nucleic Acid Probes, P. Tijssen, ed. Elsevier, N.Y. (1993).

### Detection of polypeptides

CCND3 mutations when translated into proteins can be detected by specific antibodies. Mutations in the CCND3 protein can change the antigenicity of the CCND3 protein, so that an antibody raised against a CCND3 mutant antigen (e.g. a specific peptide containing a mutation) will specifically bind the mutant CCND3 and not recognize the wild-type.

Expression level of CCND3 mutations can also be determined by examining protein expression or the protein product of CCND3 mutants. Determining the protein level involves measuring the amount of any immunospecific binding that occurs between an antibody that selectively recognizes and binds to the polypeptide of the biomarker in a sample obtained from a patient and comparing this to the amount of immunospecific binding of at least one biomarker in a control sample. The amount of protein expression of the CCND3 can be increased or reduced when compared with control expression.

A variety of techniques are available in the art for protein analysis. They include but are not limited to radioimmunoassays, ELISA (enzyme linked immunosorbent assays), "sandwich" immunoassays, immunoradiometric assays, in situ immunoassays (using e.g., colloidal gold, enzyme or radioisotope labels), western blot analysis, immunoprecipitation assays, immunofluorescent assays, flow cytometry, immunohistochemistry, confocal microscopy, enzymatic assays, surface plasmon resonance and PAGE-SDS.

### Assaying for biomarkers and CDKi treatment

Once a patient has been assayed for CCND3 status and predicted to be sensitive to a CDKi, administration of any CDKi to a patient can be effected in one dose, continuously or intermittently throughout the course of treatment. Methods of determining the most effective means and dosage of administration are well known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents may be empirically adjusted.

CCND3 mutations can be assayed for after CDKi administration in order to determine if the patient remains sensitive to the CDKi treatment. In addition, CCND3 mutations can be assayed for in multiple timepoints after a single CDKi administration. For example, an initial bolus of a CDKi is administered, a CCND3 mutation can be assayed for at 1 hour, 2 hours, 3 hours, 4 hours, 8 hours, 16 hours, 24 hours, 48 hours, 3 days, 1 week or 1 month or several months after the first treatment.

CCND3 mutations can be assayed for after each CDKi administration, so if there are multiple CDKi administrations, then assaying for CCND3 mutations for after each administration can determine continued patient sensitivity. The patient could undergo multiple CDKi administrations and then assayed for CCND3 mutations at different timepoints. For example, a course of treatment may require administration of an initial dose of CDKi, a second dose a specified time period later, and still a third dose hours after the second dose. CCND3 mutations can be assayed for at 1 hour, 2 hours, 3 hours, 4 hours, 8 hours, 16 hours, 24 hours, 48 hours, 3 days, 1 week or 1 month or several months after administration of each dose of CDKi.

Finally, different CDKi can be administered and followed by assaying for a CCND3 mutation. In this embodiment, more than one CDKi is chosen and administered to the patient. CCND3 mutation can then be assayed for after administration of each different CDKi. This assay can also be done at multiple timepoints after administration of the different CDKi. For example, a first CDKi could be administered to the patient and CCND3 mutation assayed for at 1 hour, 2 hours, 3 hours, 4 hours, 8 hours, 16 hours, 24 hours, 48 hours, 3 days, 1 week or 1 month or several months after administration. A second CDKi could then be administered and CCND3 mutation can be assayed for again at 1 hour, 2 hours, 3 hours, 4 hours, 8 hours, 16 hours, 24 hours, 48 hours, 3 days, 1 week or 1 month or several months after administration of the second CDKi.

Kits for assessing the activity of any CDKi can be made. For example, a kit comprising nucleic acid primers for PCR or for microarray hybridization for a CCND3 mutation can be used for assessing CDKi sensitivity. Alternatively, a kit supplied with antibodies for the CCND3 mutations listed in Table 2 would be useful in assaying for CDKi sensitivity.

It is well known in the art that cancers can become resistant to chemotherapeutic treatment, especially when that treatment is prolonged. Assaying for a CCND3 mutation can be done after prolonged treatment with any chemotherapeutic to determine if the cancer would be sensitive to the CDKi. For example, kinase inhibitors such as Gleevec® will strongly inhibit a specific kinase, but may also weakly inhibit other kinases. If the patient has been previously treated with another chemotherapeutic or another CDKi, it is useful to assay for a CCND3 mutation to determine if the tumor is sensitive to a CDKi. This assay can be especially beneficial to the patient if the cancer goes into remission and then re-grows or has metastasized to a different site.

### Screening for CDK inhibitors

It is possible to use CCND3 mutations to screen for other CDKi. This method comprises providing for a cell containing a CCND3 mutation from Table 2, contacting the cell with a candidate CDKi and the IC₅₀ of the treated cell is compared with a known CDKi contacting a cell containing a CCND3 mutation. For example, for cells comprising a CCND3 mutation in a PEST domain, the candidate CDKi will have an IC₅₀ greater than or equal to CDKi(1).

### EXAMPLES

### Example 1: Clustering for CCND3 mutations

The initial discovery of CCND3 mutations was made in by the clustering of CCLE sequencing data. The cell line panel is the one covered by the Cancer Cell Line Encyclopedia (CCLE) initiative (Barretina J., Caponigro G., et al. The Cancer Cell Line Encyclopedia: using preclinical models to predict anticancer drug sensitivity, Nature 2012 483(7391):603-607). A detailed genomic, genetic and pharmacologic characterization was conducted on the CCLE cell lines.

Multiplexed library for exome capture sequencing was constructed utilizing the custom SureSelect Target Enrichment System (Agilient Technologies, Santa Clara, CA). Genomic DNA from cell lines was sheared and ligated to Illumina sequencing adapters including 8 bp indexes. Adaptor ligated DNA was then size-selected for lengths between 200-350 bp and hybridized with an excess of bait in solution phase.

Barcoded exon capture libraries were then pooled and sequenced on Illumina instruments (76 bp paired-end reads). The 8 bp index was read by the instrument at the beginning of read 2 and used to assign sequencing reads to a particular sample in the downstream data aggregation pipeline.

Sequence reads were aligned to NCBI Human Reference Genome GRCh37 by BWA software (Li et al., Bioinformatics 2010 25:1754-60). Sequence reads corresponding to genomic regions that may harbor small insertions or deletions (indels) were jointly realigned using GATK local realigner (DePristo et al., Nat Genet 2011 43:491-8) as described in to improve detection of indels and to decrease the number of false positive single nucleotide variations caused by misaligned reads, particularly at the 3' end. Sites that are likely to contain indels were defined as sites of known germline indel variation from dbSNP database (Sherry et al., Nucleic Acids Res 2001 29:308-11) sites containing reads initially aligned by BWA with indels and sites adjacent to the cluster of detected nucleotide substitutions.

### Variants calling, annotation and filtering.

Nucleotide substitutions were detected with MuTect and short indels were called with Indelocator software developed at Broad Institute. Both programs were evoked in the mode that does not require matching normal DNA and identifies all variants that differ from the reference genome. Detected variants were annotated using reference transcripts derived from transcripts from the UCSC Genome Browser's "UCSC Genes" track.

### Exclusion of variants with low alleleic fraction

Allelic fraction was calculated for each detected variant in each sample as a fraction of reads that support alternative (different from the reference) allele among reads overlapping the position. To limit the effects of potential sample contamination, sub-clonal events and false positives due-to alignment artifacts only mutations with allelic fraction above 20% were used in the downstream analysis.

### Exclusion of common germline variants

Variants that have been previously reported as germline polymorphism and for which global allele frequency (GAF) in dbSNP134 or allele frequency detected in the NHLBI Exome Sequencing Project was higher than 0.1% were excluded from further analysis. Natural selection is known to be very efficient at eliminating functional deleterious mutations and usually does not allow them to reach relatively high frequency in populations; however polymorphisms at the low end of population frequency can be extremely deleterious and be identical to some of the somatic mutations. Thus few mutations identical to known germline polymorphisms, but with population frequency at or below 0.1% were retained.

### Exclusion of variants observed in panel of normals

Variants detected also in a panel of 278 whole exomes samples sequenced at the Broad as a part of 1000 Genomes Project were excluded from further analysis. In addition to removing additional germline variation this step allowed efficient removing of common false positives originating predominantly from the alignment artifacts.

### Exclusion of neutral mutations

Any amino acid substitution that creates residue observed as a wild type at the homologous position in protein's orthologs in at least two warm blooded vertebrates, was excluded from further analysis as likely being neutral. For this filtering step we used multiple amino acid alignments created by BLASTZ program and obtained from University of California Santa Cruz, Genome Browser repository.

### Identification of Cyclin D3 (CCND3) C-terminal mutations as specific for B-cell hematological malignancies

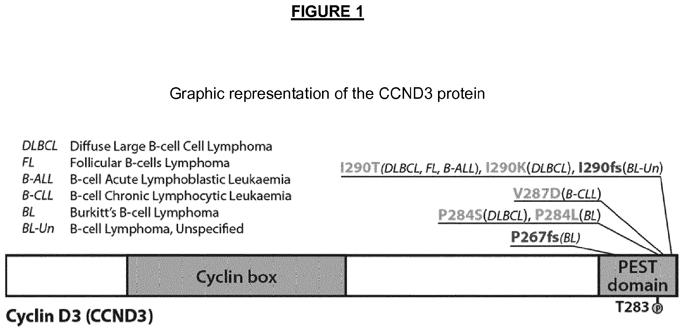

883 cell lines were categorized into 37 classes by their cell lineage and cancer type. We searched then, for mutations that have statistically significant non-random distribution across classes. We used chi-square test after normalization for widely different mutation rate in different tumors and for varying sequencing depth (that affects power to detect mutations). One of the genes that showed up as a strong hit in this analysis was CCND3. Mutations in CCND3 were present in 10 cell lines established from hematological malignancies for example, diffuse large B cell lymphoma, lymphoma, lymphocytic leukemia, acute lymphoblastic B cell leukemia and Burkitts lymphoma, but were absent in cell lines derived from solid tumors, and this data is presented in Table 2. Mutations were localized to the C-terminal domain that is known to facilitate cyclin D3 degradation and nuclear localization (Figure 1).

### Example 2: Cells containing CCND3 mutations show increased sensitivity to CDKi relative to non-mutant cells

Using the pharmacologic characterization of the CCLE cell lines, cell lines containing CCND3 mutations were tested for CDKi sensitivity. DOHH-2, NU-DHL-1, SEM, SU-DHL-4, SU-DHL-6, and SU-DHL-10 cells were purchased from Deutsche Sammlung von Mikroorganismen und Zellkulturen GmbH (DSMZ). DB and Pfeiffer cells were purchased from American Type Culture Collection (ATCC). A4/FUK cells were purchased from Health Science Research Resources Bank (HSRRB). These cells were cultured in RPMI 1640 medium (ATCC) supplemented with either 10% (A4/FUK, DB, DOHH-2, Pfeiffer, and SEM cells) or 20% (NU-DHL-1, SU-DHL-4, SU-DHL-6, and SU-DHL-10) fetal bovine serum (Gibco) and incubated at 37°C/5% CO₂. For screening, cells were seeded in 80µl of medium in 96-well plates (Costar#3904) at 10,000 (DB, DOHH-2, NU-DHL-1, SEM, SU-DHL-4, SU-DHL-6, and SU-DHL-10), 15,000 (A4/FUK) or 20,000 (Pfeiffer) cell densities and incubated overnight prior to compound addition. A4/FUK, DOHH-2, NU-DHL-1, SU-DHL-10, DB, Pfeiffer, SU-DHL-4 and SU-DHL-6 cells are diffuse large B cell lymphoma (DLBCL) cell lines. SEM cells are acute lymphoblastic B cell leukemia cells.

Compound stock (5x) was freshly prepared in the appropriate culture medium, and manually added to the plates by electronic multichannel pipette. In a minimum of three replicate wells, the number & viability of cells at the time of compound addition, as well as single agent effects after 72 hours, was assessed by quantification of cellular ATP levels via Cell Titer Glo (Promega, Madison, WI) according to the manufacturer's protocol. Day 0 subtracted- EC⁵⁰s were calculated using standard four-parametric curve fitting (XLFit, model 205).

CDKi(1)-CDKi(3) were synthesized at Novartis Pharma AG, and compound stocks were prepared in DMSO at a final concentration of 10mM. Working stocks were serially diluted in the appropriate cell culture medium in 3-fold increments to achieve final assay concentrations ranging from 10µM to 1.5nM.

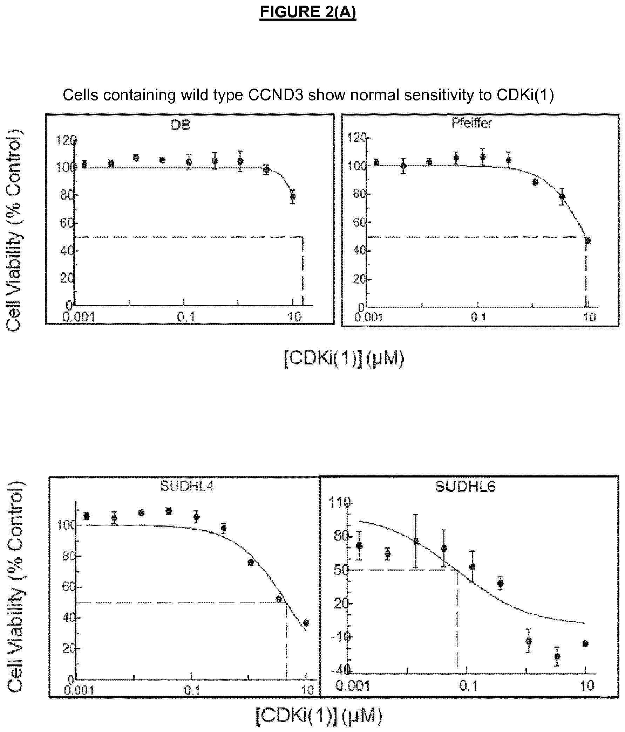

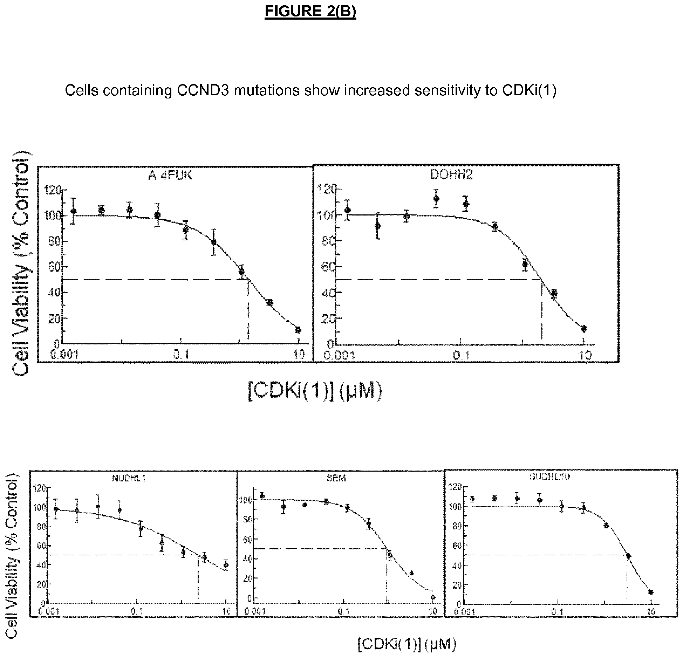

Day 0 subtracted EC⁵⁰ curves are shown for DLBCL models harboring either wild-type CCND3 as shown in Figure 2A or mutant CCND3 as shown in Figure 2B. Note that the cells containing CCND3^{WT} were sensitive to treatment with CDKi(1), and cell viability is reduced at a standard concentration. In contrast, the cells containing CCND3 mutations display a much reduced cell viability when treated with CDKi(1), demonstrating increased sensitivity to CDKi(1) when compared to the cells containing wild type CCND3.

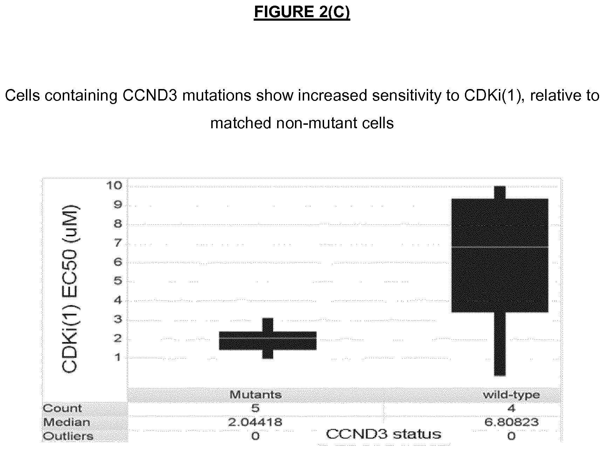

A summary of this is shown in Figure 2C. Box-plots of Day 0 subtracted EC50 values for wild-type or mutated CCND3 are shown. Central filled boxes show the lower and upper quartiles, with the group median denoted with a white line. Whiskers depict the sample minimums (lower whisker) and maximums (upper whisker), with black circles depicting sample maximums that could be considered outliers. The "Count," denotes the number of cell lines per group. A greater than 3x shift in sensitivity to CDKi(1) was observed in models with PEST-domain mutation in CCND3 as compared to wild-type.

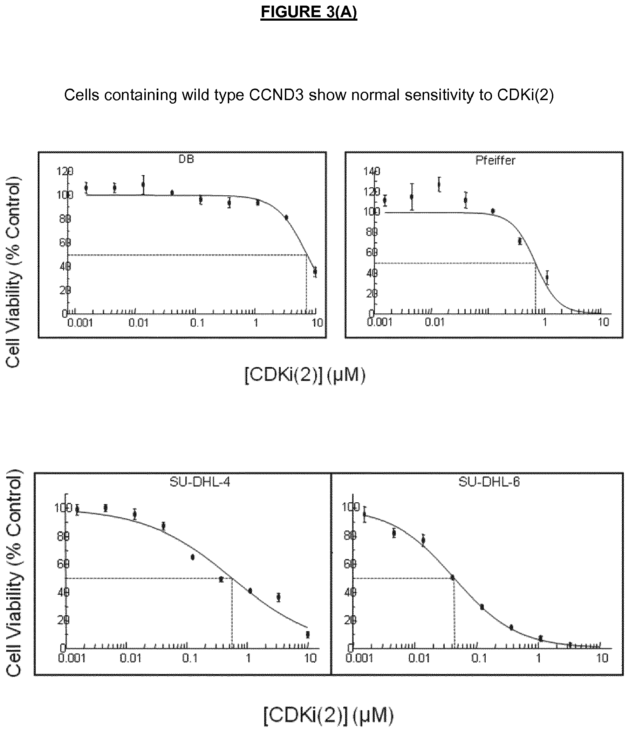

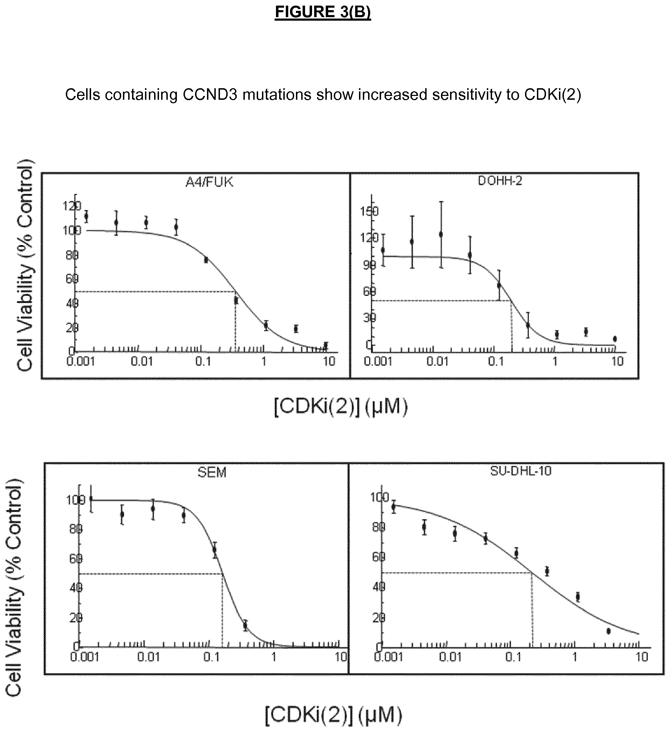

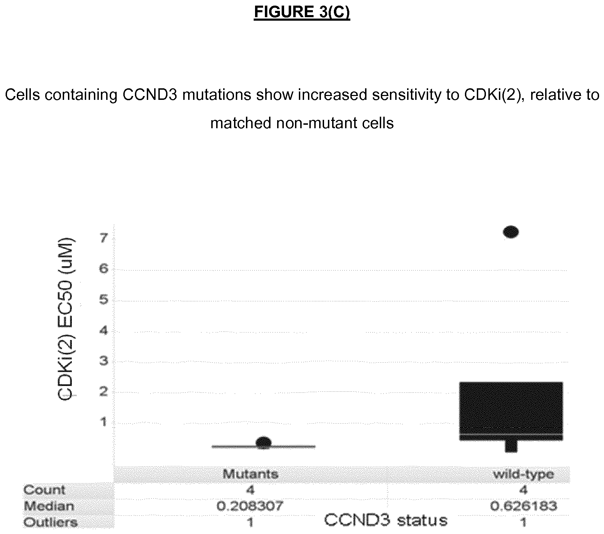

CDKi(2) is a different compound and is also known as PD0332991 (Fry et al., Mol. Cancer Ther. 2004, 3(11):1427-1438). CDKi(2) was tested in the same cell lines using the same protocol as CDKi(1) described above. The results are shown in Figure 3A and 3B. Similar to CDKi(1) the cells containing CCND3^{WT} showed normal sensitivity to treatment with CDKi(2), with reduction of cell viability at standard concentrations. When CDKi(2) was tested on CCND3 mutant cells, cell viability was reduced at much lower concentrations, indicating that these cells were more sensitive to CDKi(2) when compared with cells containing CCND3^{WT}. The box-plot in Figure 3C shows a 3X shift in sensitivity in cells with a PEST-domain mutation in CCND3 when compared to wild-type.

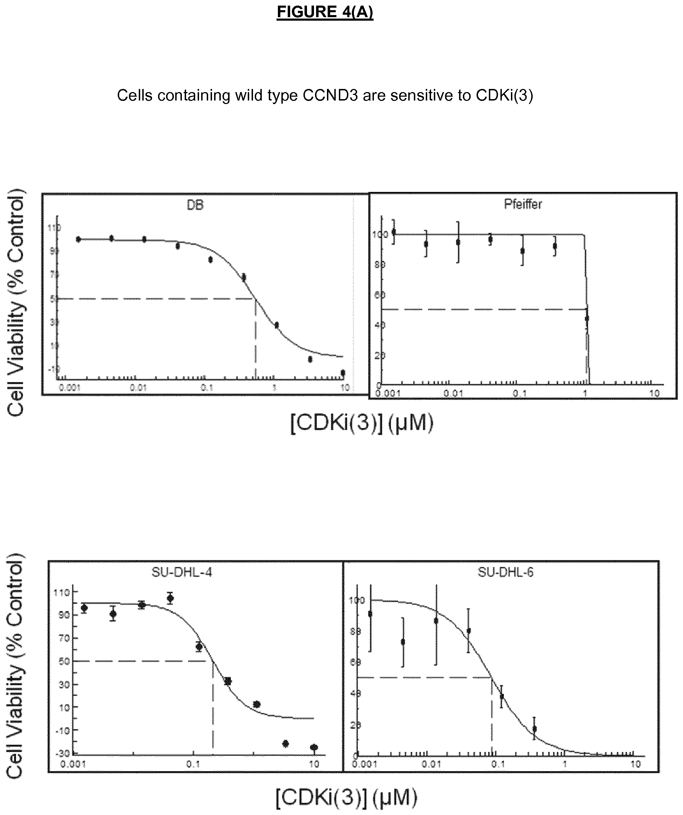

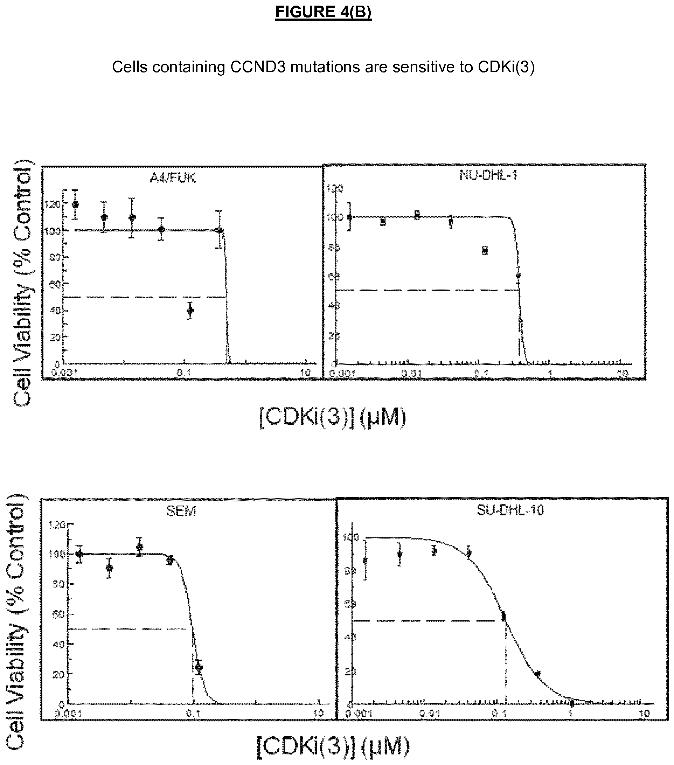

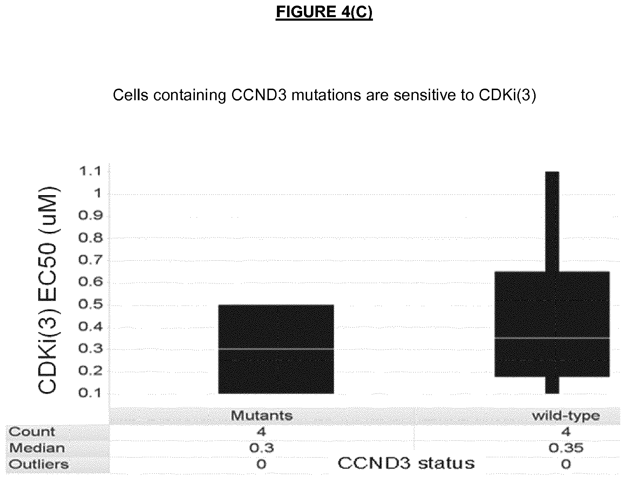

CDKi(3) is a pan CDK inhibitor, acting on all of the members of the CDK family with varying degrees of inhibition. When CDKi(3) was tested, it was found that all cell types tested (both CCND3 and wild type) were more sensitive to the pan-CDK inhibitor than to the specific CDK 4/6 inhibitors. This can be seen in the cell viability curves in Figures 4A and 4B, and there is no difference in EC⁵⁰ as shown in Figure 4C.

### Example 3: CCND3 mutations result in increased protein stability

Cells were treated with 100µg/mL cycloheximide and a minimum of 2x10⁶ cells per time point were harvested for total protein isolation. Cell lysates were prepared every 30 minutes for 2 hours, and, every 2 hours thereafter until 8 hours of treatment. DLBCL cells were lysed in buffer containing 50mM Tris, pH 7.2, 120mM NaCl, 1mM EDTA, 6mM EGTA, and 1% NP40 plus protease (Roche #05892791001, Nutley, NJ) and phosphatase inhibitors (Calbiochem #524625, Billerica, MA). After lysis, protein concentration was determined using the BCA method (Pierce #2325, Rockford, IL). Equal amounts of total protein, per cell line, were separated on a 4-12% Bis-Tris NuPAGE SDS gel (Invitrogen, Grand Island, NY) and subsequently transferred to a nitrocellulose membrane (Invitrogen, Grand Island, NY) using a dry blotting system (Invitrogen iBLOT, Grand Island, NY). Proteins were detected using the appropriate primary antibodies and an infrared dye detection system (Odyssey IRDye, LI-COR, Lincoln, NE) according to the manufacturer's protocol. Monoclonal antibodies, for Western blot analysis, are as follows: cyclin D3 (BD Biosciences #610279, Billerica, MA), Mcl-1 (Cell Signaling Technology #4572, Danvers, MA), and β-actin (Ambion #4302 Grand Island, NY). Cycloheximide was purchased from Sigma (#C4859 St. Louis, MO).

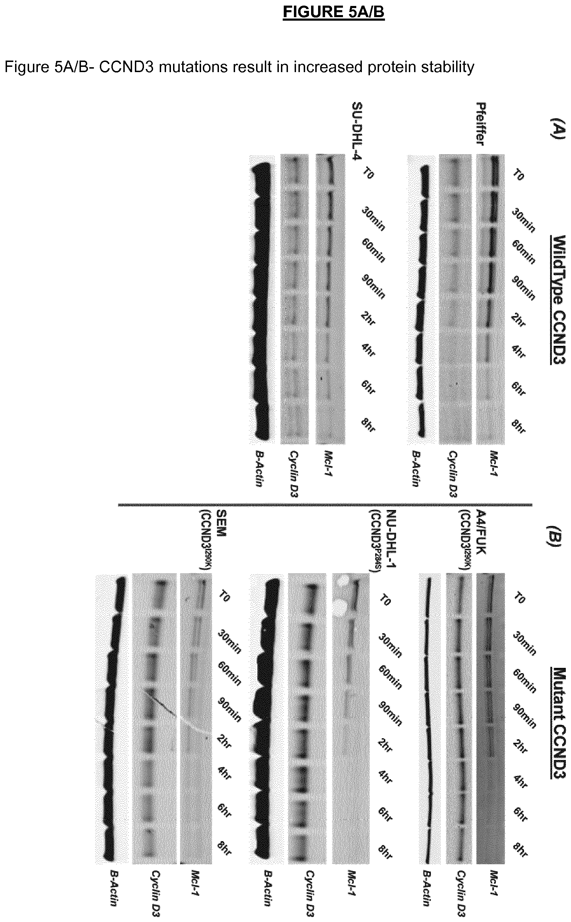

In Figure 5A, cells containing wild type CCND3 were treated with cycloheximide for the times indicated, and equal amounts of total protein were separated via SDS-PAGE. Shown are protein levels of CCND3, β-actin, and Mcl-1. Mcl-1 protein has a short half-life and was used as a control. In wild-type DLBCL cells, CCND3 is significantly depleted at 4-6 hours of treatment. In Figure 5B, CCND3 mutant cells were also treated with cyclohexamide, and displayed little or no depletion as late as 8 hours. In contrast, β-actin was equivalently stable, and Mcl-1 unstable in the different cell lines. This data suggests that a mutation in the PEST-domain of CCDN3 lead to an increase in protein stability.

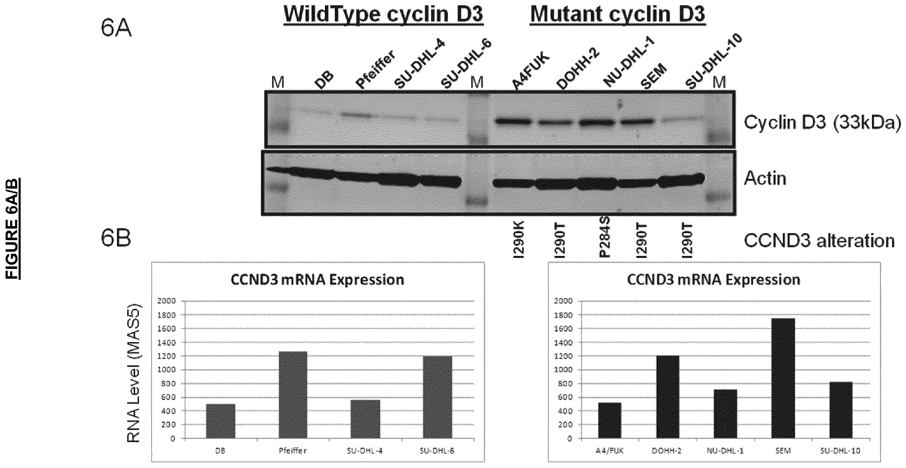

This protein stability can lead to accumulation of the CCND3 protein. Figure 6A is a Western blot demonstrating that in cells containing a CCND3 PEST domain mutation have higher protein levels of mutant CCND3. When the level of mRNA from CCND3^{WT} and CCND3 mutant cells are compared, expression levels are fairly similar, indicating that the increase in CCND3 protein is most likely due to an increase in protein stability and not an increase in expression.

Thus, without being bound to any one theory, as a CCND3 mutation does not promote or reduce CCND3 mRNA expression, a CCND3 mutation can act at the post-translational level. A representation of the CCND3 protein is shown graphically in Figure 1, and when the threonine at amino acid position 283 (T283) is phosphorlyated, this targets the CCND3 protein for ubiquitination and subsequent degradation. CCND3 has PEST domains at amino acids 256-268 and 271-291. A mutation in a PEST domain can block or reduce this phosphorylation event, thus stabilizing CCND3, resulting in increased half-life and accumulation in the cell. This stabilized CCND3 is then free to bind and activate CDK4/CDK6, which initiates uncontrolled cell proliferation, leading to cancer. The cancerous cells are driven by or are dependent on the higher levels of CDK4/6 activation, so any CDKi which binds to CDK4/6 and reduces the association of CDK4/6 with CCND3 would result in a reduction of cell proliferation despite the elevated levels of CCND3.

## Claims

1. Composition comprising a Cyclin Dependent Kinase inhibitor (CDKi) for use in treatment of cancer in a selected cancer patient population, wherein the cancer patient population is selected on the basis of showing a CCND3 mutation in a cancer cell sample obtained from said patient compared to a normal control cell sample, wherein the CCND3 mutation is in a PEST domain, wherein the cancer cell sample is selected from the group consisting of diffuse large B cell lymphoma, lymphoma, lymphocytic leukemia, acute lymphoblastic B cell leukemia and Burkitts lymphoma.

2. The composition for use according to claim 1, wherein the CCND3 mutation is at least one amino acid change in amino acids 256-268 of SEQ ID NO. 2.

3. The composition for use according to claim 1 or 2, wherein the CCND3 mutation is at least one amino acid change in amino acids 271-292 of SEQ ID NO. 2.

4. The composition for use according to claim 1 or 2, wherein the CCND3 mutation is any mutation in Table 2.

5. The composition for use according to claim 1 or 2, wherein the CCND3 mutation is selected from the group consisting of: isoleucine to lysine change at amino acid 290 (I290K), isoleucine to threonine change at amino acid 290 (I290T), proline to leucine change at amino acid 284 (P284L), proline to serine change at amino acid 284 (P284S) and valine to aspartic acid change at amino acid 287 (V287D).

6. The composition for use according to claim 1 or 2, wherein the CDKi is selected from Table 1.

## Patentansprüche

1. Zusammensetzung, umfassend einen CDK (Cyclin Dependent Kinase)-Inhibitor (CDKi) zur Verwendung bei der Behandlung von Krebs in einer ausgewählten Krebspatientenpopulation, wobei die Krebspatientenpopulation anhand des Zeigens einer CCND3-Mutation in einer von dem Patienten erhaltenen Krebszellenprobe im Vergleich zu einer normalen Kontrollzellenprobe ausgewählt wird, wobei sich die CCND3-Mutation in einer PEST-Domäne befindet, wobei die Krebszellenprobe aus der aus diffusem großzelligem B-Zell-Lymphom, Lymphom, lymphatischer Leukämie, akuter lymphoblastischer B-Zell-Leukämie und Burkitt-Lymphom bestehenden Gruppe ausgewählt ist.

2. Zusammensetzung zur Verwendung gemäß Anspruch 1, wobei es sich bei der CCND3-Mutation um wenigstens eine Aminosäureänderung in Aminosäuren 256-268 von SEQ ID NO. 2 handelt.

3. Zusammensetzung zur Verwendung gemäß Anspruch 1 oder 2, wobei es sich bei der CCND3-Mutation um wenigstens eine Aminosäureänderung in Aminosäuren 271-292 von SEQ ID NO. 2 handelt.

4. Zusammensetzung zur Verwendung gemäß Anspruch 1 oder 2, wobei es sich bei der CCND3-Mutation um eine beliebige Mutation in Tabelle 2 handelt.

5. Zusammensetzung zur Verwendung gemäß Anspruch 1 oder 2, wobei die CCND3-Mutation aus der aus Änderung von Isoleucin zu Lysin bei Aminosäure 290 (I290K), Änderung von Isoleucin zu Threonin bei Aminosäure 290 (I290T), Änderung von Prolin zu Leucin bei Aminosäure 284 (P284L), Änderung von Prolin zu Serin bei Aminosäure 284 (P284S) und Änderung von Valin zu Asparaginsäure bei Aminosäure 287 (V287D) bestehenden Gruppe ausgewählt ist.

6. Zusammensetzung zur Verwendung gemäß Anspruch 1 oder 2, wobei der CDKi aus Tabelle 1 ausgewählt ist.

## Revendications

1. Composition comprenant un inhibiteur de kinase cycline-dépendante (CDKi) pour utilisation dans le traitement du cancer dans une population de patients atteints du cancer choisie, la population de patients atteints du cancer étant choisie sur la base du fait qu'elle présente une mutation de CCND3 dans un échantillon de cellule cancéreuse obtenu auprès dudit patient par rapport à un échantillon de cellule témoin normal, la mutation de CCND3 étant dans un domaine PEST, l'échantillon de cellule cancéreuse étant choisie dans le groupe constitué d'un lymphome diffus à grandes cellules B, un lymphome, la leucémie lymphocytaire, la leucémie à cellules B lymphoblastique aiguë et le lymphome de Burkitt.

2. Composition pour utilisation selon la revendication 1, la mutation de CCND3 étant au moins un changement d'acide aminé dans les acides aminés 256 à 268 de SEQ ID NO. 2.

3. Composition pour utilisation selon la revendication 1 ou 2, la mutation de CCND3 étant au moins un changement d'acide aminé dans les acides aminés 271 à 292 de SEQ ID NO. 2.

4. Composition pour utilisation selon la revendication 1 ou 2, la mutation de CCND3 étant une mutation quelconque dans le tableau 2.

5. Composition pour utilisation selon la revendication 1 ou 2, la mutation de CCND3 étant choisie dans le groupe constitué de : un changement d'isoleucine en lysine au niveau de l'acide aminé 290 (I290K), un changement d'isoleucine en thréonine au niveau de l'acide aminé 290 (I290T), un changement de proline en leucine au niveau de l'acide aminé 284 (P284L), un changement de proline en sérine au niveau de l'acide aminé 284 (P284S) et un changement de valine en acide aspartique au niveau de l'acide aminé 287 (V287D).

6. Composition pour utilisation selon la revendication 1 ou 2, le CDKi étant choisi dans le tableau 1.


### SEQUENCE LISTING

<110> Novartis Institutes for Biomedical Science
<120> BIOMARKERS ASSOCIATED WITH CDK INHIBITORS
<130> PAT055187-US-PSP
<140> US61/669534
   <141> 2012-07-09
<160> 2
<170> PatentIn version 3.5
<210> 1
   <211> 2011
   <212> DNA
   <213> Homo sapiens
<400> 1
   cgcgccccgc gctctccggc ccgtcgcctg ccttgggact cgcgagcccg cactcccgcc 60
   ctgcctgttc gctgcccgag tatggagctg ctgtgttgcg aaggcacccg gcacgcgccc 120
   cgggccgggc cggacccgcg gctgctgggg gaccagcgtg tcctgcagag cctgctccgc 180
   ctggaggagc gctacgtacc ccgcgcctcc tacttccagt gcgtgcagcg ggagatcaag 240
   ccgcacatgc ggaagatgct ggcttactgg atgctggagg tatgtgagga gcagcgctgt 300
   gaggaggaag tcttccccct ggccatgaac tacctggatc gctacctgtc ttgcgtcccc 360
   acccgaaagg cgcagttgca gctcctgggt gcggtctgca tgctgctggc ctccaagctg 420
   cgcgagacca cgcccctgac catcgaaaaa ctgtgcatct acaccgacca cgctgtctct 480
   ccccgccagt tgcgggactg ggaggtgctg gtcctaggga agctcaagtg ggacctggct 540
   gctgtgattg cacatgattt cctggccttc attctgcacc ggctctctct gccccgtgac 600
   cgacaggcct tggtcaaaaa gcatgcccag acctttttgg ccctctgtgc tacagattat 660
   acctttgcca tgtacccgcc atccatgatc gccacgggca gcattggggc tgcagtgcaa 720
   ggcctgggtg cctgctccat gtccggggat gagctcacag agctgctggc agggatcact 780
   ggcactgaag tggactgcct gcgggcctgt caggagcaga tcgaagctgc actcagggag 840
   agcctcaggg aagcctctca gaccagctcc agcccagcgc ccaaagcccc ccggggctcc 900
   agcagccaag ggcccagcca gaccagcact cctacagatg tcacagccat acacctgtag 960
   ccctggagag gccctctgga gtggccacta agcagaggag gggccgctgc cacccacctc 1020
   cctgcctcca ggaaccacac cacatctaag cctgaagggg cgtctgttcc cccttcacaa 1080
   agcccaaggg atctggtcct acccatcccc gcagtgtgca ctaaggggcc cggccagcca 1140
   tgtctgcatt tcggtggcta gtcaagctcc tcctccctgc atctgaccag cagcgccttt 1200
   cccaactcta gctgggggtg ggccaggctg atgggacaga attggataca tacaccagca 1260
   ttccttttga acgccccccc ccacccctgg gggctctcat gttttcaact gccaaaatgc 1320
   tctagtgcct tctaaaggtg ttgtcccttc tagggttatt gcatttggat tggggtccct 1380
   ctaaaattta atgcatgata gacacatatg agggggaata gtctagatgg ctcctctcag 1440
   tactttggag gcccctatgt agtccgtgct gacagctgct cctagaggga ggggcctagg 1500
   cctcagccag agaagctata aattcctctt tgctttgctt tctgctcagc ttctcctgtg 1560
   tgattgacag ctttgctgct gaaggctcat tttaatttat taattgcttt gagcacaact 1620
   ttaagaggac gtaatggggt cctggccatc ccacaagtgg tggtaaccct ggtggttgct 1680
   gttttcctcc cttctgctac tggcaaaagg atctttgtgg ccaaggagct gctatagcct 1740
   ggggtggggt catgccctcc tctcccattg tccctctgcc ccatcctcca gcagggaaaa 1800
   tgcagcaggg atgccctgga ggtggctgag cccctgtcta gagagggagg caagccctgt 1860
   tgacacaggt ctttcctaag gctgcaaggt ttaggctggt ggcccaggac catcatccta 1920
   ctgtaataaa gatgattgtg aaataaaact ggctttggct tcttggaaaa aaaaaaaaaa 1980
   aaaaaaaaaa aaaaaaaaaa aaaaaaaaaa a 2011
<210> 2
   <211> 292
   <212> PRT
   <213> Homo sapiens
<400> 2
   Met Glu Leu Leu Cys Cys Glu Gly Thr Arg His Ala Pro Arg Ala Gly
   1               5                   10                  15
   Pro Asp Pro Arg Leu Leu Gly Asp Gln Arg Val Leu Gln Ser Leu Leu
               20                  25                  30
   Arg Leu Glu Glu Arg Tyr Val Pro Arg Ala Ser Tyr Phe Gln Cys Val
           35                  40                  45
   Gln Arg Glu Ile Lys Pro His Met Arg Lys Met Leu Ala Tyr Trp Met
       50                  55                  60
   Leu Glu Val Cys Glu Glu Gln Arg Cys Glu Glu Glu Val Phe Pro Leu
   65                  70                  75                  80
   Ala Met Asn Tyr Leu Asp Arg Tyr Leu Ser Cys Val Pro Thr Arg Lys
                   85                  90                  95
   Ala Gln Leu Gln Leu Leu Gly Ala Val Cys Met Leu Leu Ala Ser Lys
               100                 105                 110
   Leu Arg Glu Thr Thr Pro Leu Thr Ile Glu Lys Leu Cys Ile Tyr Thr
               115                 120                 125
   Asp His Ala Val Ser Pro Arg Gln Leu Arg Asp Trp Glu Val Leu Val
       130                 135                 140
   Leu Gly Lys Leu Lys Trp Asp Leu Ala Ala Val Ile Ala His Asp Phe
   145                 150                 155                 160
   Leu Ala Phe Ile Leu His Arg Leu Ser Leu Pro Arg Asp Arg Gln Ala
                   165                 170                 175
   Leu Val Lys Lys His Ala Gln Thr Phe Leu Ala Leu Cys Ala Thr Asp
               180                 185                 190
   Tyr Thr Phe Ala Met Tyr Pro Pro Ser Met Ile Ala Thr Gly Ser Ile
           195                 200                 205
   Gly Ala Ala Val Gln Gly Leu Gly Ala Cys Ser Met Ser Gly Asp Glu
       210                 215                 220
   Leu Thr Glu Leu Leu Ala Gly Ile Thr Gly Thr Glu Val Asp Cys Leu
   225                 230                 235                 240
   Arg Ala Cys Gln Glu Gln Ile Glu Ala Ala Leu Arg Glu Ser Leu Arg
                   245                 250                 255
   Glu Ala Ser Gln Thr Ser Ser Ser Pro Ala Pro Lys Ala Pro Arg Gly
               260                 265                 270
   Ser Ser Ser Gln Gly Pro Ser Gln Thr Ser Thr Pro Thr Asp Val Thr
           275                 280                 285
   Ala Ile His Leu
       290